# EUROPEAN PATENT SPECIFICATION

(11) **EP 0 553 667 B1**
(45) Date of publication and mention of the grant of the patent: **05.06.2002**
(21) Application number: 93100587.0
(22) Date of filing: 15.01.1993
(51) Int. Cl.: A61K 38/16, C07K 14/00

(54) **Soluble LDL receptor, its production and use**
Löslicher LDL-Rezeptor, seine Herstellung und Verwendung
Récepteur à LDL-soluble, sa production et son utilisation

(30) Priority: 19.01.1992 IL 100696; 23.08.1992 IL 102915
(43) Date of publication of application: 04.08.1993
(73) Proprietor: YEDA RESEARCH AND DEVELOPMENT COMPANY LIMITED, Rehovot 76100 (IL)
(72) Inventor: Rubinstein, Menachem, Givat Shmuel (IL); Novick, Daniela, Rehovot (IL); Tal, Nathan, Rehovot (IL); Fischer, Dina G., REHOVOT (IL)
(74) Representative: Grünecker, Kinkeldey, Stockmair & Schwanhäusser Anwaltssozietät

(56) References cited:
- EP-A- 0 485 961
- WO-A-83/01383
- GB-A- 2 161 487

## Description

The present invention relates to a process for the production of a protein exhibiting antiviral activity and to a purified protein obtainable by this process. Further, the present invention relates to a DNA molecule encoding the recombinant protein, to an expression vector and to a cell line. Finally, the present invention relates to a process for the production of recombinant soluble low density lipoprotein (LDL) receptor and to pharmaceutical compositions containing it.

### BACKGROUND OF THE INVENTION

Interferons (IFN) are inducible proteins that are produced by various cells and induce an antiviral state in animal cells. There are three major types of IFNs, distinguished by their antigenic properties: α, β and gamma. IFN-α and IFN-β are related proteins of 166 or 165 amino acid residues that are induced by viruses or nucleic acids and are produced by cells from various tissues, including immune cells. IFN-gamma is a protein of 130-143 amino acid residues which is produced by mitogen-activated T-cells and by large granular lymphocytes. The production of IFN is usually transient and it stops shortly after the inducer disappears. For a recent review of these issues, see Taylor S.L. and Grossberg S.E., 1990, Virus Research, 15, 1-26.

In addition to the three well-characterized types of interferons, there are several reports describing partially characterized species of interferons. A group of IFN-α-like (IFN-α1) genes and pseudogenes, also known as class II IFN-α or IFN-omega was discovered and reported (Revel, M., 1984, in "Antiviral Drugs and Interferon: The Molecular Basis of their Activity", Y. Becker (ed.), Martinus Neijhoff Publ., Boston, pp. 357-434; Capon, D.J. et al., 1985, Molec. Cell. Biol., 5, 768-779; Hauptmann, R. and Swetly, P., 1985, Nuc.Acid.Res., 13, 4739-4749). These are virus-induced interferons having about 172 amino acid residues which are present in the natural mixture of human IFN-α produced , by leukocytes (Adolf, G.R., 1990, Virology, 175, 410-417).

Treatment of human peripheral blood mononuclear leukocytes with a mitogen resulted in production of IFN-gamma and a novel IFN-like substance named IFN-δ (Wilkinson, M. and Morris, A., 1983, Biochem.Biophys.Res.Comm. 111, 498-503). IFN-δ was found to be acid resistant and active only on human fibroblasts having chromosome -21 trisomy and not on WISH cells. It was antigenically distinct from the three known IFN types.

Acid-labile alpha-interferons were described in several publications. An acid-labile IFN-α was induced in cultures of lymphocytes from individuals who have recently received influenza vaccine, by stimulation in vitro with the influenza virus (Balkwill, F.R. et al., 1983, J.Exp.Med., 157, 1059-1063). This type of IFN was neutralized by anti-IFN-α serum and was active on Mandin Darby Bovine Kidney (MDBK) cells. The presence of such acid-labile alpha-type IFN in sera of patients with systemic lupus erythematosus was reported (Klippel, J.H. et al, 1985, Annals Rheum.Disease, 44, 104-108). An acid-labile IFN-α was produced similarly to IFN-α by Sendai virus induction of human peripheral leukocytes (Matsuoka, H. et al., 1985, J. Gen.Virol., 66, 2491-2494). Acid-labile IFN-α was spontaneously produced in cultures of peripheral blood mononuclear cells (Fischer, D.G. and Rubinstein, M., 1983, Cellular Immunology, 81, 426-434).

Another type of IFN called IFN-epsilon was produced by epithelial cells exposed to virus. It was produced together with IFN-β but was active on epithelial cells and not on other cell types (Jarvis, A.P. and Kosowsky, D.I., 1984, US Patent No. 4,614,651).

Among other cytokines, TNF, IL-6 and IL-1 were reported to exhibit antiviral activity (Mestan, J. et al., 1986, Nature, 323, 816-819; Wong, G.H.W. and Goedell, D., 1986, Nature, 323, 819-822; Billiau, A., 1987, Antiviral Research, 8, 55-70). TNF is produced only by immune cells and it was suggested that IL-1 and TNF exert their antiviral activity by inducing the production of IFN-β (Billiau, A., op.cit.).

Several interferon-induced proteins have been identified and some of them were shown to be instrumental in the induction of the antiviral state by IFNs. The best studied one is (2'-5') oligo adenylate synthetase, an intracellular enzyme which polymerizes ATP into ppp(A2'-5'p)nA, where n is preferably 2 or 3, but may be as long as 15 (Kerr, I.M. and Brown, R.E., 1978, Proc.Natl.Acad. Sci.USA, 75, 256-260). Such oligomers activate a latent ribonuclease (RNASE-F) which degrades ribosomal RNA and polysomes, thereby inhibiting viral and cellular protein synthesis. Another IFN-induced intracellular enzyme is a 2'-5'phosphodiesterase which may remove the CCA terminus of tRNA, thereby leading to inhibition of protein synthesis (Schmidt, A. et al., 1979, Proc.Natl.Acad.Sci. USA, 76, 4788-4792). A third known IFN-induced intracellular enzyme is a 70 Kd protein kinase which phosphorylates the Initiation Factor eIF-2, thereby leading to inhibition of the initiation of mRNA translation into proteins (Ohtsuki, K. et al., 1980, Nature, 287, 65-67). Other IFN-induced intracellular proteins include the nuclear IFN-Responsive Factors (IRF-1 and IRF-2) which regulate IFN-responsive genes; metalothionein, a 56 Kd protein of unknown function in the IFN-induced antiviral state; Factor B of the alternative complement system and the murine Mx gene product, which is responsible for resistance to influenza (Reviewed in Taylor, I.L. and Grossberg, S.E., 1990, Virus Research, 15, 1-26). Other IFN-induced cell associated polypeptides were identified on 2-D gels following IFN treatment and [³⁵S]-methionine pulsing, but these proteins were not further characterized in terms of their structure and function (Veil, J. et al., 1983, Nature, 301, 437-439). Several cell surface interferon-induced proteins were identified, including class I and II MHC antigens, IgG, Fc receptor and cytoskeletal components (Reviewed in Revel, M., 1984, in "Antiviral Drugs and Interferons: The Molecular Basis of their Activity", Y. Becker (ed.), pp. 357-434, Martinus Neijhoff Publ., Boston).

Additional IFN-induced proteins that were secreted into the medium have been disclosed in the literature, such as β2-microglobulin, a shedded component of the cell-surface class I MHC antigens (Dolei, A.F. et al., 1981, Antiviral Res., 1, 367-373), and plasminogen activator and lymphotoxin, which were induced in lymphocytes by IFN (Jones, C.M. et al., 1982, J.IFN.Res., 2, 377-386; Wallach, D. and Hahn, T., 1983, Cellular Immunol., 76, 390-396). IFN-gamma-treated monocytes released TNF which enhanced the overall antiviral effect (Gerrard, T. et al., 1989, J.IFN.Res., 9, 115-124). IFN-gamma-induced proteins of molecular weight 30,000 (extracellular) and 25,000 (intracellular) were described (Luster A.D. et al., 1988, J.Biol.Chem., 263, 12036-12043), but their role was not determined.

Although many IFN-induced proteins have been disclosed, none of them is related to a soluble LDL receptor. The existence of a soluble LDL receptor as a separate protein has not been so far disclosed. The full size low density lipoprotein receptor (LDLR) is a transmembrane glycoprotein which is not soluble in the absence of detergents. It consists of 822 amino acid residues and exhibits a molecular weight of 164,000. Its only known function is to internalize LDL and VLDL. Structurally it consists of several domains, some of which are shared with other proteins. The N-terminal ligand-binding domain is made of 292 amino acid residues arranged in 7 cysteine-rich imperfect repeats.

This domain is followed by a region homologous to the EGF precursor (400 amino acid residues), a region of 58 amino acid residues rich in 0-linked sugars, a single trans-membrane domain of 22 amino acid residues and a cytoplasmic domain of 50 amino acid residues (Schneider W.J. et al., J. Biol.Chem. 257, 2664-2673, 1982; Yamamoto T. Et al., Cell 39, 27-38, 1984). EP-A-0586094 discloses a truncated human LDLR protein consisting of amino acids 1 to 354 of the mature LDL-receptor, the construction of a gene encoding said LDLR and its expression in a baculovirus system. However, there is no mention of antiviral properties of the LDL receptor.

US patent 4,745,060 discloses the isolation and characterization of bovine and human LDLR DNA s as well as recombinant transfer vectors comprising said DNA s and their use in methods for diagnosing familial hypercholesterolemia.

### SUMMARY OF THE INVENTION

It has been found that when human fibroblasts or epithelial cells are treated with an interferon, a protein showing antiviral activity is produced and accumulates in the supernatant of the cell cultures. The present invention provides a process for the production of a protein exhibiting antiviral activity, comprising treating a culture of interferon-inducible cells with an interferon, incubating the culture, harvesting the culture supernatant, and purifying the protein from fractions of the supernatant exhibiting antiviral activity.

The invention also provides a puritied protein obtainable by such process. This protein has now been purified to homogeneity and was identified as the soluble extracellular region of LDL receptor.

The present invention thus provides a soluble extracellular domain of the mature LDL receptor protein, its muteins and fused proteins, and their salts, functional derivatives and active fractions, with the proviso that said protein is not the full length LDL receptor. The antiviral activity of the receptor protein may be conveniently determined, for example, in a system consisting of WISH amnion cells and vesicular stomatitis virus (VSV) as a challenge.

The invention also provides a soluble LDL receptor corresponding to the extracellular portion (750 amino acid residues) of the LDL receptor, which is purified to homogeneity with respect to proteinaceous impurities.

The invention relates especially to the soluble extracellular domain of the mature LDL receptor comprising at least but not exclusively the ligand binding domain of the mature LDL receptor, and, more specifically, to a protein having an amino acid sequence corresponding to amino acid residue 4 to 292 of the mature LDL receptor.

The invention also relates to a soluble extracellular domain of the mature LDL receptor, including the amino acid sequence as shown in the following: ASP-ARG-PRO-GLU-ARG-ASN-GLU-PHE-GLN- () -GLN-ASP-GLY-LYS-PRO, wherein "()" is an unidentified amino acid.

The invention further concerns recombinant DNA molecules comprising the nucleotide sequence coding for said protein or for its active muteins or fused proteins, with the proviso that the DNA molecule does not consist of nucleotides 1 to 323 of the human LDL receptor cDNA sequence, expression vehicles comprising them and host cells transformed therewith, and to a process for producing a recombinant soluble LDL receptor, its active muteins or fused proteins, by culturing said transformant cells in a suitable culture medium and isolating the soluble LDL receptor.

The soluble LDL receptor of the invention, its active muteins, fused proteins, and their salts, functional derivatives and active fractions, are for use as active ingredients of pharmaceutical compositions to protect mammals against viral infections.

### BRIEF DESCRIPTION OF THE FIGURES

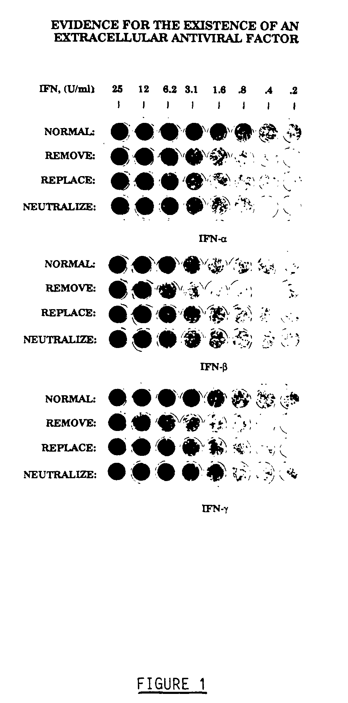

Figure 1 shows evidence for the existence of antiviral activity which is not the added interferon. The protective effect of various interferons against the cytopathic effect of vesicular stomatitis virus (VSV) on human amnion WISH cells, is determined under a variety of conditions. The assay was performed in 96-well plates and each row represents a serial two-fold dilution of IFN. The final concentration of IFN in the first column from left is 25 IU/ml. From top: Row 1: IFN-α added and VSV added after 24 hrs; Row 2: IFN-α added, cells washed after 24 hrs, fresh IFN-α added and VSV added; Row 3: IFN-α added, neutralizing anti-IFN-α antibody added after 24 hrs, followed by addition of VSV. Rows 4 to 6 and 7 to 9 are replicates of the first three rows, except that IFN-β and anti-IFN-β antibody, and IFN-gamma and anti-IFN-gamma antibody were used, respectively.
Figure 2 shows the elution pattern of the proteins and antiviral activity from a TSK®-DEAE anion exchange column.
Figure 3 shows the elution pattern of proteins and antiviral activity from a hydroxyapatite Biogel HTP column.
Figure 4 shows the elution pattern of the proteins and antiviral activity from a Superformance® TMAE-65-S anion exchange HPLC column.
Figure 5 shows the elution pattern of proteins and antiviral activity from a phenyl sepharose hydrophobic interaction column.
Figure 6 shows the elution pattern of protein and antiviral activity from a reversed-phase Aquapore® RP-300 HPLC column.
Figure 7 shows the elution pattern of protein and antiviral activity from rechromatography on a reversed-phase HPLC column.
Figure 8 shows sodium dodecyl sulfate - polyacrylamide gel electrophoretic (SDS-PAGE) analysis of various fractions obtained in the last step of the purification procedure. A 13% acrylamide gel was used and stained with silver.
   The lanes are: 1-7, aliquots (400 µl) of fractions 10-16 respectively from RP-300 chromatography (Figure 7) which were concentrated by ultrafiltration; 8, control sample buffer; 9, molecular weight markers, indicated on the left side.
Figure 9 shows the elution pattern of antiviral activity from a Superose 12 size exclusion HPLC column.
Figure 10 shows the original output of the protein microsequencer. Fractions 10-12 of RP-HPLC (Fig. 7) 0.4 ml each, were pooled, concentrated by ultrafiltration and the resulting sample (1µg) was subjected to protein microsequence analysis.
Figure 11 shows the computer output of the search for the sequence obtained by the protein microsequencer shown in Figure 10.
Figure 12 shows the total antiviral activity of various fractions from the immunoaffinity chromatography of partially purified soluble LDL receptor on monoclonal antibody C7 column. The bars are: load - column load; effl. - effluent (unbound fraction); el.1-4 - elutions 1-4.
Figure 13 shows a Western immunoblotting analysis of the various fractions from the immunoaffinity chromatography (shown in Figure 12). The proteins were electroblotted to nitrocellulose and visualized with monoclonal antibody C7 and ¹²⁵I-protein A. Lanes: 1. Molecular weight markers; 2. load; 3. unbound; 4. wash; 5. elution 2; 6. elution 3; 7. monoclonal antibody C7.
Figure 14 shows a dose response induction of cell surface LDL receptor in WISH cells by various interferons. Cell were incubated with interferons for 19 has in medium containing 2% fetal calf serum and the level of cell surface LDL receptor was then determined by monoclonal antibody C7 and ¹²⁵I-protein A.

### DETAILED DESCRIPTION OF PREFERRED EMBODIMENTS

It has been found that interferon-treated cells secrete into the culture medium an antivirally active protein which is not an interferon. This protein is not neutralized by antibodies directed against all three types of human interferons (both NIH standards and neutralizing monoclonal antibodies). The protein was identified as comprising the extracellular ligand binding domain of the LDL receptor.

The soluble LDL receptor is secreted into the culture medium by mammalian cells that enter into an antiviral state in response to any interferon. Examples are fibroblast cells or epithelial cells derived from amniotic fluid, e.g. U cells, WISH cells.

In contrast to interferons, the soluble LDL receptor does not induce in the cells an antiviral state, but is antiviral by itself. Thus, cells treated simultaneously with the soluble LDL receptor and virus will not lyse, while cells treated with virus alone or simultaneously with virus and IFN-gamma, will be infected and lyse after 12 hours. This shows that IFN-gamma gives no immediate protection to the cells against virus since it takes about 10 hrs until the antiviral state is established in cells treated with IFN-gamma. In contrast, the soluble LDL receptor protects the cells immediately when added to them.

When examined by size exclusion chromatography, the soluble LDL receptor has an apparent molecular weight of about 40,000.

For the production of the soluble LDL receptor, suitable cells grown in culture are treated with an IFN in a suitable medium and then incubated for some hours at 37°C, thus producing the soluble LDL receptor which is secreted into the medium and can be isolated from the supernatant. Suitable cells are those capable of entering into an antiviral state in response to an interferon. Similar results were obtained with IFN-α, IFN-β and IFN-gamma, but IFN-gamma is preferably used because it does not exhibit antiviral activity under the conditions of the antiviral assay for the soluble LDL receptor (simultaneous addition of the soluble LDL receptor and challenge virus to the cell culture).

In a preferred embodiment of the invention, human WISH cells are treated with IFN-gamma in MEM supplemented with a serum substitute and then further incubated at 37°C. The highest titer of the soluble LDL receptor was obtained after 17 hrs. The supernatant of the cells containing the soluble LDL receptor is then harvested and concentrated by known methods, such as by ultra-filtration or by dialysis against semi-solid polyethylene glycol 20,000. The concentrated supernatant is then purified by chromatographic procedures.

In a preferred embodiment, purified soluble LDL receptor is produced by a process comprising the following steps:
a. growing human WISH cells in culture to confluency, inducing the cells with 30 U/ml IFN-gamma in a serum-free medium and, after about 17 hrs, harvesting the culture supernatant;
b. concentrating said supernatant about 30-fold, by e.g. ultrafiltration with a membrane of molecular weight cut-off of about 10,000.
c. subjecting said concentrated supernatant of step (b) to anion exchange chromatography to obtain partially purified active fractions of the antiviral factor;
d. applying said partially purified fraction from step (c) to chromatography on a hydroxyapatite column to obtain partially purified fractions of the antiviral factor;
e. applying said partially purified fraction from step (d) to anion exchange HPLC to obtain partially purified fractions of the antiviral factor;
f. applying said partially purified fractions from step (e) to hydrophobic interaction chromatography to obtain partially purified fractions of the antiviral factor; and
g. applying said partially purified active fractions from step (f) to reversed phase high pressure liquid chromatography (HPLC) at about neutral pH to obtain partially purified antiviral factor.
h. repeating step g. to obtain homogenous antiviral factor, defined by its ability to inhibit the cytopathic effect (CPE) exerted by vesicular stomatitis virus (VSV) on human WISH cells.

The ion exchange chromatography of step (c) is preferably performed on a TSK®-DEAE column (Tosoh Corp., Japan) at pH about 8 eluted with increasing salt concentration. The hydroxyapatite chromatography is preferably perfomed on a Biogel HTP column (BioRad, USA) at pH 6.8 with phosphate buffers as eluants. The anion exchange HPLC is preferably performed on a Superformance® TMAE column in a manner similar to the TSK-DEAE step. The hydrophobic interaction chromatography is preferably performed on a phenyl sepharose column eluted with decreasing salt concentration. The reversed phase HPLC is preferably performed on an Aquapore® RP300 column, at pH 7.5, with a gradient of acetonitrile.

In another preferred embodiment soluble LDL receptor is purified by a process in which one or more of the chromatographic steps is replaced by immunoaffinity chromatography on an anti LDL receptor monoclonal antibody column, e.g. a process comprising steps a, b and c as described in the aforementioned embodiment and then performing a step of immunoaffinity chromatography on a column of a monoclonal antibody directed against the soluble LDL receptor.

The monoclonal antibody can preferably be the one made by hybridoma C7 (ATCC, CRL 1691 and the soluble receptor is eluted at a high pH. The partially purified soluble LDL receptor is loaded onto the columns at neutral pH, the column is washed with 0.5M NaCl at neutral pH and the soluble LDL receptor is eluted in an enhanced state of purity with 50 mM Na₂CO₃ (pH 11) and immediately neutralized.

Preferably, in all steps of the purification the chromatography is monitored by measuring the protein concentration (absorbance at 280 nm or relative fluorescence following "on-line" reaction of representative aliquots with fluorescamine). The antiviral activity in each fraction is determined by inhibition of the VSV-induced CPE in WISH cells according to the bioassay described herein.

In the following the soluble extracellular domain of the mature LDL receptor according to the invention will be referred to as "soluble LDL receptor".

As used herein the term "muteins" refers to analogs of the soluble LDL receptor in which one or more of the amino acid residues of the natural soluble LDL receptor are replaced by different amino acid residues or are deleted, or one or more amino acid residues are added to the natural sequence of the soluble LDL receptor, without changing considerably the antiviral activity of the resulting product. These muteins are prepared by known synthesis and/or by site-directed mutagenesis techniques, or any other known technique suitable therefor.

The term "fused protein" refers to a polypeptide comprising the soluble LDL receptor or a mutein thereof fused with another protein which has an extended residence time in body fluids. The soluble LDL receptor may thus be fused to another protein, polypeptide or the like, e.g. an immunoglobulin or a fragment thereof.

The term "salts" herein refers to both salts of carboxyl groups and to acid addition salts of amino groups of the soluble LDL receptor, muteins and fused proteins thereof. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids such as, for example, hydrochloric acid or sulfuric acid, and salts with organic acids such as, for example, acetic acid or oxalic acid.

"Functional derivatives" as used herein cover derivatives of the soluble LDL receptor and its fused proteins and muteins, which may be prepared from the functional groups which occur as side chains on the residues or the N- or C-terminal groups, by means known in the art, and are included in the invention as long as they remain pharmaceutically acceptable, i.e. they do not destroy the activity of the protein and do not confer toxic properties on compositions containing it. These derivatives may, for example, include polyethylene glycol side-chains which may mask antigenic sites and extend the residence of the soluble LDL receptor in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl or threonyl residues) formed with acyl moieties. The term "functional derivative" also includes proteins which have an amino acid sequence longer or shorter than the sequence determined, as long as the protein still has the ability to inhibit viral infection.

As "active fractions" of the soluble LDL receptor, its fused proteins and its muteins, the present invention covers any fragment or precursors of the polypeptide chain of the protein molecule alone or together with associated molecules or residues linked thereto, e.g. sugar or phosphate residues, or aggregates of the protein molecule or the sugar residues by themselves, provided said fraction has the ability to inhibit viral infection.

This invention further concerns DNA molecules comprising the nucleotide sequence encoding the soluble LDL receptor, fused proteins, muteins or active fractions thereof, replicable expression vehicles containing said DNA molecules, hosts transformed therewith and protein produced by expression of such transformed hosts. The term "DNA molecules" includes genomic DNA, cDNA, synthetic DNA and combinations thereof.

This invention further concerns DNA molecules comprising a nucleotide sequence encoding a protein having an amino acid sequence corresponding to the amino acid sequence of the ligand binding domain of the mature LDL receptor, fused proteins, muteins or active fractions thereof, with the proviso that the DNA molecule does not consist of nucleotides 1 to 323 of the human LDL receptor cDNA sequence. The invention also concerns replicable expression vehicles containing said DNA molecules, hosts transformed therewith and protein produced by expression of such transformed hosts. The term "DNA molecules" includes genomic DNA, cDNA, synthetic DNA and combinations thereof. Preferably, the DNA molecule encodes a soluble LDL receptor having an amino acid sequence corresponding to amino acid residue 4 to 292 of the mature LDL receptor. In a further embodiment, the DNA molecule encodes a soluble LDL receptor including the amino acid sequence as follows:

ASP-ARG-PRO-GLU-ARG-ASN-GLU-PHE-GLN- () -GLN-ASP-GLY-LYS-PRO.

The invention also concerns a DNA molecule which hybridises to any of the above DNA molecules and encodes a soluble LDL receptor, with the proviso that the DNA molecule does not consist of nucleotides 1 to 323 of the human LDL receptor cDNA sequence.

The production of the recombinant soluble LDL receptor may be carried out by different techniques. According to one approach, the known cDNA of the soluble LDL receptor is taken from plasmid pLDLR-2 (Yamamoto et al., op cit.). The DNA is subjected to site directed mutagenesis with appropriate oligonucleotides so that a termination codon and a polyadenylation site are inserted after codon 292 of the mature LDL receptor. This construct is then inserted into appropriately constructed expression vectors by techniques well known in the art (see Maniatis et al., op cit.). Double-stranded cDNA is linked to plasmid vectors by homopolymeric tailing or by restriction linking involving the use of synthetic DNA linkers or blunt-ended ligation techniques. DNA ligases are used to ligate the DNA molecules and undesirable joining is avoided by treatment with alkaline phosphatase.

The production of a fused protein comprising the ligand binding domain of the LDL receptor and eg. the constant region of IgGz heavy chain may be carried out as follows: the DNA of pLDLR is subjected to site-directed mutagenesis with appropriate oligonucleotides so that a unique restriction site is introduced immediately after codon 292 of the mature LDL receptor. A plasmid bearing the constant region of IgG₂ heavy chain, e.g. pRKCO4₂Fc₁ (Byrn R.A. et al., 1990 Nature (London) 344, 667-670) is subjected to similar site-directed mutagenesis to introduce the same unique restriction site as close as possible to Asp 216 of IgG₁ heavy chain in a way that allows translation in phase of the fused protein. A dsDNA fragment consisting of 5' untranslated sequences and encoding the leader and about the first 295 amino acids of LDL receptor is prepared by digestion of the mutated pLDL receptor at the EcoRI and the unique restriction sites. The mutated pRKCD4₂Fc₁ is similarly digested to generate a large fragment containing the plasmid and the IgG₁ sequences. The two fragments are then ligated to generate a new plasmid encoding a polypeptide consisting of about the N-terminal 295 amino acids of LDL receptor and about 227 C-terminal amino acids of IgG₁ heavy chain (hinge region and CH2 and CH3 domains). The DNA encoding the fused protein may be isolated from the plasmid by digestion with EcoRI and then inserted into an efficient expression vector.

In order to be capable of expressing the soluble LDL receptor, its muteins or the fused proteins, an expression vector should comprise also specific nucleotide sequences containing transcriptional and translational regulatory information linked to the DNA coding for the desired protein in such a way as to permit gene expression and production of the protein. First, in order for the gene to be transcribed, it must be preceded by a promoter recognizable by RNA polymerase, to which the polymerase binds and thus initiates the transcription process. There are a variety of such promoters in use, which work with different efficiencies (strong and weak promoters). They are different for prokaryotic and eukaryotic cells.

The promoters that can be used in the present invention may be either constitutive, for example, the int promoter of bacteriophage lambda, the bla promoter of the β-lactamase gene of pBR322, and the CAT promoter of the chloramphenicol acetyl transferase gene of pPR325, etc., or inducible, such as the prokaryotic promoters including the major right and left promoters of bacteriophage lambda (P_{L} and P_{R}), the trp, recA, lacZ, lacI, ompF and gal promoters of E. coli, or the trp-lac hybrid promoter, etc. (Glick, B.R. (1987) J. Ind. Microbiol. 1:277-282).

Besides the use of strong promoters to generate large quantities of mRNA, in order to achieve high levels of gene expression in prokaryotic cells, it is necessary to use also ribosome-binding sites to ensure that the mRNA is efficiently translated. One example is the Shine-Dalgarno sequence (SD sequence) appropriately positioned from the initiation codon and complementary to the 3'-terminal sequence of 16S RNA.

For eukaryotic hosts, different transcriptional and translational regulatory sequences may be employed, depending on the nature of the host. They may be derived from viral sources, such as adenovirus, bovine papilloma virus, Simian virus, or the like, where the regulatory signals are associated with a particular gene which has a high level of expression. Examples are the TK promoter of Herpes virus, the SV40 early promoter, the yeast ga14 gene promoter, etc. Transcriptional initiation regulatory signals may be selected which allow for repression and activation, so that expression of the genes can be modulated.

The DNA molecule comprising the nucleotide sequence coding for the soluble LDL receptor of the invention or its fragments or muteins or fused proteins thereof, and the operably linked transcriptional and translational regulatory signals is inserted into a vector which is capable of integrating the desired gene sequences into the host cell chromosome. In order to be able to select the cells which have stably integrated the introduced DNA into their chromosomes, one or more markers which allow for selection of host cells which contain the expression vector is used. The marker may provide for prototrophy to an auxotropic host, biocide resistance, e.g., antibiotics, or heavy metals, such as copper, or the like. The selectable marker gene can either be directly linked to the DNA gene sequences to be expressed, or introduced into the same cell by cotransfection. Additional elements may also be needed for optimal synthesis of single chain binding protein mRNA. These elements may include splice signals, as well as transcription promoters, enhancers, and termination signals. cDNA expression vectors incorporating such elements include those described by Okayama, H., (1983) Mol. Cel. Biol. 3:280.

In a preferred embodiment, the introduced DNA molecule will be incorporated into a plasmid or viral vector capable of autonomous replication in the recipient host. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Preferred prokaryotic vectors include plasmids such as those capable of replication in E. coli, for example, pBR322, ColEl, pSC101, pACYC 184, etc. (see Maniatis et al., op.cit.); Bacillus plasmids such as pC194, pC221, pT127, etc. (Gryczan, T., "The Molecular Biology of the Bacilli", Academic Press, NY (1982), pp. 307-329); Streptomyces plasmids including pIJ101 (Kendall, K.J. et al., (1987) J. Bacteriol. 169:4177-4183); Streptomyces bacteriophages such as φC31 (Chater, KF. et al., in "Sixth International Symposium on Actinomycetales Biology", Akademiai Kaido, Budapest, Hungary (1986), pp. 45-54), and Pseudomonas plasmids (John, J.F., et al. (1986) Rev. Infect. Dis. 8:693-704), and Izaki, K. (1978) Jpn. J. Bacteriol. 33:729-742).

Preferred eukaryotic plasmids include BPV, vaccinia, SV40, 2-micron circle, etc., or their derivatives. Such plasmids are well known in the art (Botstein, D., et al. (1982) Miami Wint.Symp. 19:265-274; Broach, JR., in "The Molecular Biology of the Yeast Saccharomyces: Life Cycle and Inheritance", Cold Spring Harbor Laboratory, Cold Spring Harbor, NY, pp. 445-470 (1981); Broach, J.R., (1982) Cell 28:203-204; Bollon, D.P., et al. (1980) J. Clin. Hematol. Oncol. 10:39-48; Maniatis, T., in "Cell Biology: A Comprehensive Treatise, Vol. 3: Gene Expression", Academic Press, NY, pp. 563-608 (1980)).

Once the vector or DNA sequence containing the construct(s) has been prepared for expression, the expression vector may be introduced into an appropriate host cell by any of a variety of suitable means, such as transformation, transfection, lipofection, conjugation, protoplast fusion, electroporation, calcium phosphate precipitation, direct microinjection, etc.

Host cells to be used in this invention may be either prokaryotic or eukaryotic. Preferred prokaryotic hosts include bacteria such as E. coli, Bacillus, Streptomyces, Pseudomonas, Salmonella, Serratia, etc. The most preferred prokaryotic host is E. coli. Bacterial hosts of particular interest include E. coli K12 strain 294 (ATCC 31446), E. coli X1776 (ATCC 31537), E. coli W3110 (F⁻, lambda⁻, prototropic (ATCC 27325)), and other enterobacterium such as Salmonella typhimurium or Serratia narcescens and various Pseudomonas species. Under such conditions, the protein will not be glycosylated. The prokaryotic host must be compatible with the replicon and control sequences in the expression plasmid.

However, since the soluble LDL receptor is a cysteine rich protein, eukaryotic hosts are preferred over prokaryotic hosts. Preferred eukaryotic hosts are mammalian cells, e.g., human, monkey, mouse and chinese hamster ovary (CHO) cells, because they provide post-translational modifications to protein molecules including correct folding, correct disulfide bond formation as well as glycosylation at correct sites. Also yeast cells and insect cells can carry out post-translational peptide modifications including high mannose glycosylation. A number of recombinant DNA strategies exist which utilize strong promoter sequences and high copy number of plasmids which can be utilized for production of the desired proteins in yeast and in insect cells. Yeast cells recognize leader sequences on cloned mammalian gene products and secrete peptides bearing leader sequences.

After the introduction of the vector, the host cells are grown in a selective medium, which selects for the growth of vector-containing cells. Expression of the cloned gene sequence(s) results in the production of the soluble LDL receptor, a fusion protein, or a mutein or a fragment thereof. The expressed protein is then isolated and purified by any conventional procedure involving extraction, precipitation, chromatography, electrophoresis, or the like, or by affinity chromatography, using anti-soluble LDL receptor monoclonal antibodies (eg. hybridoma C7, Belsiegel U. et al., J.Biol.Chem., 256, 11923-11931, 1981) immobilized on a gel matrix contained within a column. Crude preparations containing said recombinant soluble LDL receptor are passed through the column whereby the soluble LDL receptor will be bound to the column by the specific antibody while the impurities will pass through. After washing, the protein is eluted from the gel at a high pH, eg. pH 11.

The soluble LDL receptor and its muteins, fused proteins and their salts, functional derivatives, and active fractions thereof are indicated for the treatment of viral diseases in mammals.

The present invention further relates to pharmaceutical compositions comprising a pharmaceutically acceptable carrier and the soluble LDL receptor of the invention or its active muteins, fused proteins and their salts, functional derivatives or active fractions thereof, either as the sole active ingredient or in combination with other antiviral agents, e.g. interferons. These compositions may be used against viral diseases. The way of administration can be via any of the accepted modes of administration for similar agents and will depend on the condition to be treated, e.g., intravenously or intramuscularly or subcutaneously, in case of systemic viremia, or local injection or topical application in case of a localized infection, or continuously by infusion, etc.

The pharmaceutical compositions of the invention are prepared for administration by mixing the soluble LDL receptor its derivatives, alone or together with other antiviral agents, with physiologically acceptable carriers, stabilizers and excipients, and prepared in dosage form, e.g. by lyophilization in dosage vials. The amount of active compound to be administered will depend on the route of administration, the disease to be treated and the condition of the patient. Local injection, for instance, will require a lower amount of the protein on a body weight basis than will intravenous infusion in case of systemic viremia.

The invention will now be illustrated by the following non-limiting examples.

### EXAMPLES

### Example 1: Preliminary identification of the antiviral activity of the soluble LDL receptor

The presence of an unknown antiviral factor in the supernatant of IFN-induced cells was shown by several experiments.

The antiviral effect was determined by the virus cytopathic effect (CPE) reduction assay (Rubinstein, S. et al (1981) J. Virol. 37:755-758) using human WISH cells and VSV as a challenge. Initial studies were performed with IFN-α, IFN-β and IFN-gamma and similar results were obtained with all three IFNs.

In the experiment described in Figure 1, and summarized in Table 1, human amnion WISH cells (ATCC CCL-25) were seeded in a 96-well microtiter plate. A few hours later, serial twofold dilutions (from right to left), starting with 50 U/ml of IFN-α, IFN-β and IFN-gamma in MEM (Minimal Essential Medium) supplemented with 10% fetal bovine serum, were applied to monolayers of the cells in rows (from top) 1-3, 4-6 and 7-9, respectively, and the cells were incubated overnight at 37°C. The medium in rows 1, 5 and 9 was not removed. The medium in rows 2, 6 and 10 was aspirated, the cell monolayers were washed once and fresh medium was added. In rows 3, 7 and 11, the medium was replaced with fresh dilutions of the respective IFNs, and in rows 4, 8 and 12, neutralizing antibodies specific to IFN-α, IFN-β (NIH standards) and to IFN-gamma (monoclonal antibody 166.5, described in Novick et al., (1983) EMBO 3(2), p. 1527) were added respectively. VSV in growth medium was applied to the cells in all wells. The infected cultures were further incubated overnight and then stained with crystal violet, in order to better evaluate visually the extent of the virus cytopathic effect.

**TABLE 1**

| **The role of antiviral factor in IFN action** | | | | |
|---|---|---|---|---|
| Row of Fig. 1 | Treatment⁽¹⁾ | 50% CPE at dilution | units/ml⁽²⁾ | % of activity |
| 1 | IFN-α 24 hrs, standard assay | 400 | 1000 | 100 |
| 2 | IFN-α 24 hrs, wash | 100 | 250 | 25 |
| 3 | IFN-α 24 hrs, replace IFN | 200 | 250 | 25 |
| 4 | IFN-α 24 hrs, neutralize | 300 | 375 | 37 |
| 5 | IFN-β 24 hrs, standard assay | 400 | 1000 | 100 |
| 6 | IFN-β 24 hrs, wash | 200 | 500 | 50 |
| 7 | IFN-β 24 hrs, replace IFN | 400 | 1000 | 100 |
| 8 | IFN-β 24 hrs, neutralize | 400 | 1000 | 100 |
| 9 | IFN-γ 24 hrs, standard assay | 800 | 1000 | 100 |
| 10 | IFN-γ 24 hrs, wash | 200 | 250 | 25 |
| 11 | IFN-γ 24 hrs, replace IFN | 400 | 500 | 50 |
| 12 | IFN-γ 24 hrs, neutralize | 800 | 1000 | 100 |

| | | | | |
|---|---|---|---|---|
| ⁽¹⁾ In the standard assay, (rows 1, 5 and 9) IFN was added in serial twofold dilutions (from right to left in Fig. 1) to monolayers of WISH cells in 96-well plates. After 24 hrs, VSV was added and 18 hrs later the cells were fixed and stained. In other cases, washing (rows 2, 6 and 10), replacement with IFN in fress medium (rows 3, 7 and 11) or addition of anti-IFN neutralizing antibodies (rows 4, 8 and 12), were done immediately prior to challenge with VSV. | | | | |
| ⁽²⁾ The titers of IFN standards (rows 1, 5 and 9) were designated as 1000 U/ml. | | | | |

The dilutions at which NIH standard IFNs (rows 1, 5 and 9) gave 50% CPE were first determined. As seen in Figure 1, rows 2, 6 and 10, washing off the cells after 24 hrs and prior to VSV challenge, significantly reduced the potency of all three IFNs. Replacement of the medium with fresh dilutios of IFN reduced the level of protection with IFN-α (row 3) and IFN-gamma (row 1), but not with IFN-β (row 7). Addition of neutralizing anti-IFN antibodies to the growth medium after 24 hrs exposure to IFN, had only a small effect on the activity of IFN-α (row 4) and no effect on the activity of IFN-β and IFN-gamma (rows 8 and 12, respectively).

Since IFNs induce antiviral state in cells after interaction with specific cell surface receptors, they can be removed once the antiviral state is established and the cells will remain protected against the virus. Therefore removal of IFN after the antiviral state was established, is not expected to affect the apparent IFN potency. However, in the above experiment, it was shown that the apparent antiviral potency of a given IFN sample was significantly lower if the culture medium was replaced prior to challenge with virus (rows 2, 6 and 10). Even if the medium was replaced with fresh IFN, both in the case of IFN-α (row 3) and IFN-gamma (row 11) the level of protection was lower than without replacement. Hence the culture medium had a non-interferon component that protects the cells from virus infection.

Incubation of the cells after application of IFN and addition of anti-IFN neutralizing antibodies prior to virus challenge without replacement of the growth medium, lowered the antiviral activity of the IFN-α only slightly (row 4) and not the activity of IFN-β or IFN-gamma (rows 8 and 12, respectively). These results suggest that the reduced activity observed when the IFN-containing medium was removed, is not due to removal of the IFN molecules, but rather to removal of other molecules which are required to achieve full antiviral protection.

The antiviral enhancing activity in the medium, was herein demonstrated in vitro in the IFN CPE reduction assay. It is possible that the active extracellular component, plays also a role in vivo in the IFN antiviral activity. When IFN is used in a systemic disease where secreted components are eliminated continuously, administration of such a factor might greatly enhance the effect of IFN.

### Example 2: Production and purification of the antiviral protein

### 2.1 Production of crude antiviral protein

Human amnion cells WISH (ATCC CCL-25) were grown to confluency on Fibracell discs (Sterilin, U.K.) in spinner flasks, in a medium consisting of MEM supplemented with 10% fetal calf serum (FCS). At confluency, the medium was discarded and the discs washed several times with serum-free MEM. The cells were then incubated in MEM (1.3 1) supplemented with a protein-free serum substitute ADC-1 (1:50, Biological Industries, Beit Haemek, Israel), Hepes 20 mM, Insulin 0.2 µg/ml and IFN-gamma (30 U/ml). Incubation was continued for 17 hrs at 37°C. The culture medium was then collected, spun (5000 xg, 15 min) and the supernatant was collected and kept under sterile conditions at 4°C for short periods (up to 24 hrs), or at -20°C until used. The cell culture could be continuously used for production by adding more protein-free medium and IFN-gamma.

### 2.2 Concentration of crude antiviral factor

The antiviral factor can be concentrated either by dialysis against polyethylene glycol 20,000 or by ultrafiltration. Crude cell supernatant (1.5 l) of step 2.1 above was concentrated about 30-fold by ultrafiltration in a Minitan® unit (Millipore, USA) with a polysulfone membrane of molecular weight 10,000 cut off (PTGC Minitan® plate). The crude retentate was washed with sodium borate buffer, 20 mM, pH 8 (Buffer A) and brought to a volume of about 50 ml. This material was used immediately or kept frozen at -20°C until used.

### 2.3 Chromatography on TSK®-DEAE

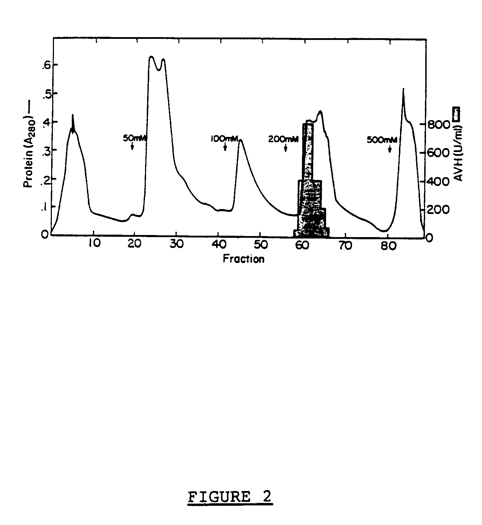

A TSK®-DEAE column (2.5x33 cm, Tosoh, Japan) was equilibrated with Buffer A. Concentrated antiviral factor from Minitan® step 2.2 above was applied to the column at a flow rate of 8 ml/min. The column was then washed with Buffer A and eluted stepwise with 50, 100, 200 and 500 mM NaCl in Buffer A. Fractions of 12 ml were collected and subjected to bioassay. The column was monitored by absorbance at 280 nm (Fig. 2). The protein peak eluted with 200 mM NaCl, contained antiviral activity when tested in the presence of neutralizing anti IFN-gamma monoclonal antibody No. 166-5. It was pooled and concentrated to a volume of about 20 ml by ultrafiltration on a YM-10 membrane (MW cut off 10,000, Amicon USA). The material was kept at -20° until used.

### 2.4 Hydroxyapatite chromatography

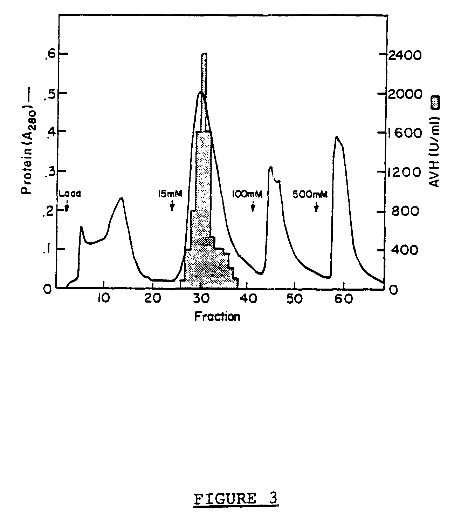

A hydroxyapatite biogel HTP column (2.5 X 4 cm, BioRod, USA) was equilibrated with water. The concentrated 0.2m NaCl protein peak of step 2.3 (166 mg) was loaded on the Biogel HTP column at a flow rate of 2 ml/min. The column was washed with water and eluted with 15 mM sodium phosphate pH 6.8. Fractions of 2 ml were collected and tested for antiviral activity. The column was monitored by absorbance at 280 nm (Fig. 3). The antiviral activity was found in the 15 mM Na phosphate eluate, pooled and concentrated by ultrafiltration.

### 2.5 Anion exchange HPLC

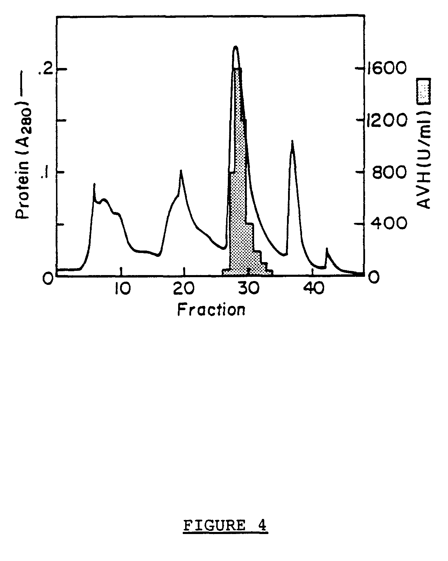

An anion exchange HPLC column (Superformance® - TMAE - 650S, E. Merck, Germany) was pre-equilibrated with Buffer A. The concentrated pool containing 73 mg protein from step 2.4 was spun (10,000xg, 5 min.) and the supernatant was applied to the column at a flow rate of 1 ml/min. The column was washed with Buffer A and then eluted stepwise by 50, 100, 200 and 500 mM NaCl in Buffer A. Fractions of 2.5 ml were collected and assayed for antiviral activity. The column was monitored at 280 nm (Fig. 4). The activity eluted in the 200 mM NaCl fraction. This fraction was pooled and kept at -20°c until used.

### 2.6 Hydrophobic interaction chromatography

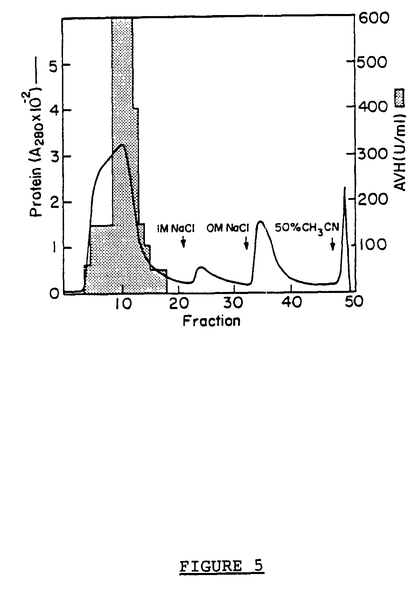

A phenyl sepharose column (1.5x6.5 cm, Pharmacia, Sweden) was equilibrated with 1.5 M NaCl in Buffer A. The 200mM Na phosphate protein peak of step 2.5 (10 mg) was brought to 1.5 M NaCl and loaded (1 ml/min.) on the phenyl sepharose column. The column was washed with 1.5M NaCl in Buffer A and unbound protein peak was collected. The column was eluted stepwise with 1M NaCl in Buffer A, Buffer A and 50% CH₃CN/50% Buffer A. Antiviral activity was obtained in the unbound (1.5 M NaCl) fraction. The column was monitored at 280 nM (Fig. 5).

### 2.7 Reversed phase HPLC

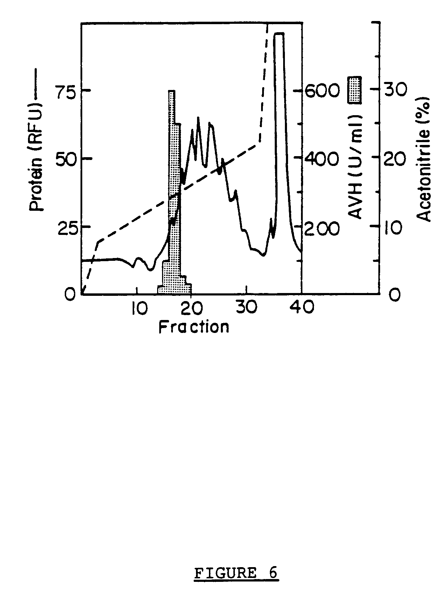

The unbound pooled fraction from step 2.6 (1.2 mg) was loaded on an Aquapore® RP-300 RP-HPLC column (4.6x30 mm) that was preequilibrated with 20mM Hepes buffer pH 7.5. The column was washed and eluted at a flow rate of 0.5ml/min by an acetonitrile gradient in the same buffer. Fractions of 1 ml were collected and tested for antiviral activity. The antiviral activity eluted at 14% acetonitrile and was associated with a protein peak. However this peak was not completely resolved from adjacent peaks (Fig. 6). The column was monitored by a fluorescamine-based post-column reaction system (Stein S. and Moschera J., 1981, Methods in Enzymology, 79:7-16).

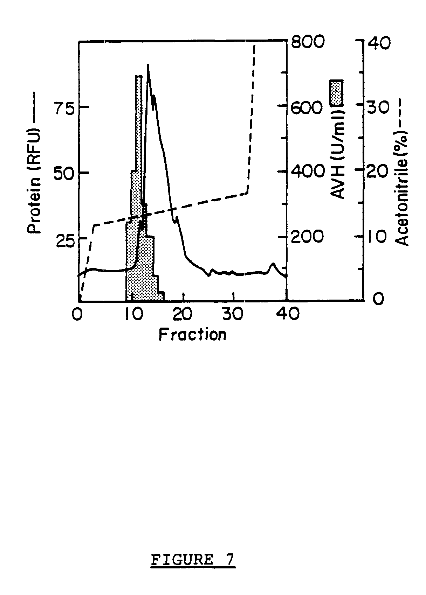

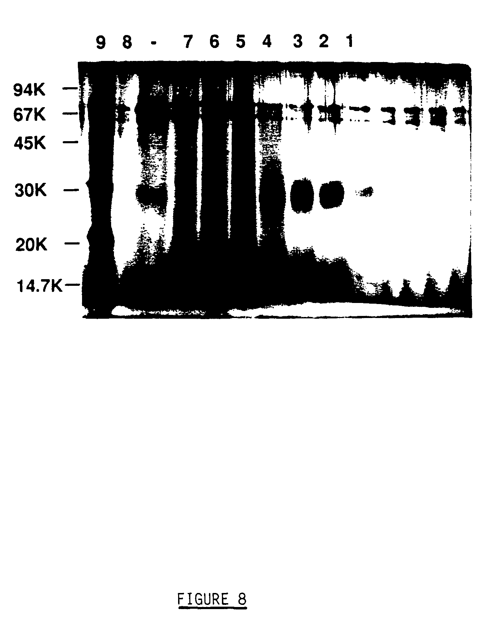

Active fractions (56µg) were pooled, diluted twofold with 20mM Hepes, pH 7.5 and rechromatographed on the Aquapore® RP-300 column (Fig. 7). Aliquots (400µl) of each fraction were concentrated by ultrafiltration and subjected to polyacrylamide (13%) gel electrophoresis in the presence of sodium dodecyl sulfate and β-mercaptoehtanol (SDS-PAGE). The protein bands were visualized by silver staining. The lanes are: lane 1-7, fractions 10-16 respectively of the HPLC; lane 8, control sample buffer; lane 9, molecular weight markers, indicated on the left side (Fig. 8).

### Example 3: Characterization of the antiviral factor

### 3.1 Bioassay of antiviral activity

The assay is similar to the cytopathic effect (CPE) inhibition assay which is used for measuring IFN activity (Rubinstein S. et al., (1981) J. Virol. 37: 755-758). The antiviral activity is calibrated against NIH reference standard of human IFN-β. It can also be calibrated with IFN-α standard, but not with IFN-gamma, which does not protect the cells from virus under these assay conditions. The following procedure is used:

Confluent monolayers of WISH cells are prepared on day 1, seeding 45,000 cells/well in 100µl of MEM supplemented with 10% FCS, in 96-flat bottom well plates. The plates are incubated at 37°C in 5% CO₂. On day 2, samples of the antiviral factor are diluted twofold serially in a separate plate (100 µl). Neutralizing monoclonal anti-IFN-gamma antibody (166-5) sufficient for neutralizing 1000 U/ml of IFN-gamma is added to each well and the solutions are transferred to the plate with the WISH cells, followed immediately by challenge with an appropriate amount (see below) of stock VSV (50 µl). The plates are incubated overnight at 37°C. The assay is calibrated against standard IFN-β. On day 3, about 20 hrs following virus challenge, the cytopathic effect in control wells is observed microscopically. When it is ≥80%, the plates are drained and the monolayer is stained with crystal violet (0.5% in 70% Aq. methanol), washed with plenty of tap water and the end points are determined by observation under the microscope. An appropriate amount of VSV for this assay is the dilution of stock VSV which, when added to a serial twofold dilution of standard IFN-β under the assay conditions, will give 50% CPE at IFN-β dilution of about 3-6 U/ml after 20-24 hrs ofincubation.

### 3.2 The antiviral factor is a protein

Three experiments were performed in order to demonstrate that the antiviral factor is a protein.
a. Molecular weight > 10,000, as shown by the ability to concentrate the antiviral activity by dialysis against PEG 20,000 and by ultrafiltration on membranes with cut-off of 10,000 Da, indicating that the antiviral factor is a macro-molecule.
b. Heat lability. Active fraction from a Mono Q anion exchange step (same as step 2.5 of example 2) was heated in a 100°C bath for 10 min and then tested for antiviral activity. No activity remained following this treatment. See Table 2.
c. Trypsin sensitivity. Active fraction from the Mono Q® anion exchange step was incubated with TPCK-treated trypsin (Worthington) at 5:1 protein:enzyme ratio, respectively, overnight at room temperature. Control fraction was similarly kept without trypsin and control trypsin was made as well. It was found that 66% of the antiviral activity was lost by trypsin treatment and hence, it was concluded that the antiviral factor is trypsin sensitive. See Table 2.
d. The antiviral factor is not an interferon. Interferon is defined as a protein which induces in cells an antiviral state which persists even after removal of the interferon. Cells were incubated with antiviral factor-containing medium from the Mono Q® step for 24 hrs, the medium was removed, the cells were washed and challenged with VSV. No protection from VSV was observed. Hence it was concluded that the antiviral factor is not an interferon and its mechanism of action is different from that of an interferon.
e. The antiviral factor is probably not a degradative enzyme. The site and mode of action of the antiviral factor is still not known. In order to clarify these questions, further studies must be carried out with different viruses. One simple experiment was performed to test whether the factor is an enzyme that degrades viruses, eg. a proteolytic enzyme. For this purpose, VSV was incubated with serial twofold dilutions of the factor for different time periods at 37°C. Bovine MDBK cells were then added to the wells and the extent of CPE was determined. It was found that VSV gave the same extent of CPE when mixed with the cells immediately after addition of the antiviral factor or when pre-incubated with the antiviral factor for 5 hrs at 37°C. Hence it was concluded that the antiviral factor is probably not a virus degrading enzyme.

**TABLE 2:**

| **Sensitivity of the antiviral factor to heat and trypsin** | | |
|---|---|---|
| | Activity | |
| Sample | U/ml | % |
| Stock (Mono Q®) antiviral factor, overnight, room temp. | 75 | 100 |
| Stock antiviral factor + trypsin, overnight, room temp. | 25 | 33 |
| Stock antiviral factor (Mono Q®), room temp., 10 min. | 65 | 100 |
| Stock antiviral factor, 100°C, 10 min. | 0 | 0 |

### 3.3 Size exclusion chromatography of the antiviral factor

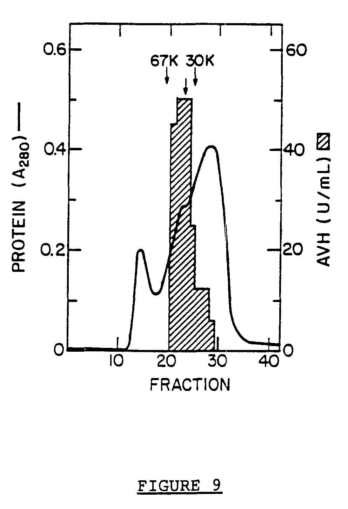

Antiviral factor from the TSK®-DEAE step (0.4 ml) was fractionated on a size exclusion column (Superose® 12, 1x30 cm, Pharmacia) in phosphate buffered saline under near physiological conditions. The column was pre-equilibrated and eluted with phosphate buffered saline at a flow rate of 0.5 ml/min. Fractions of 1 ml were collected and antiviral activity was measured in each fraction. The column was monitored at 280 nm (Fig. 9). The column was calibrated with bovine serum albumin (67K) and carbonic anhydrase (30K) as molecular weight markers. It was found that the antiviral activity eluted as a peak of apparent molecular weight 40,000. However, this peak was rather broad.

### 3.4 Species specificity of the antiviral factor

The antiviral factor was found to be active on human WISH cells. It was also found to be active on bovine MDBK cells and murine L cells. Hence it was concluded that the factor is not species specific.

### 3.5 Protein sequence analysis and identification of the antiviral factor

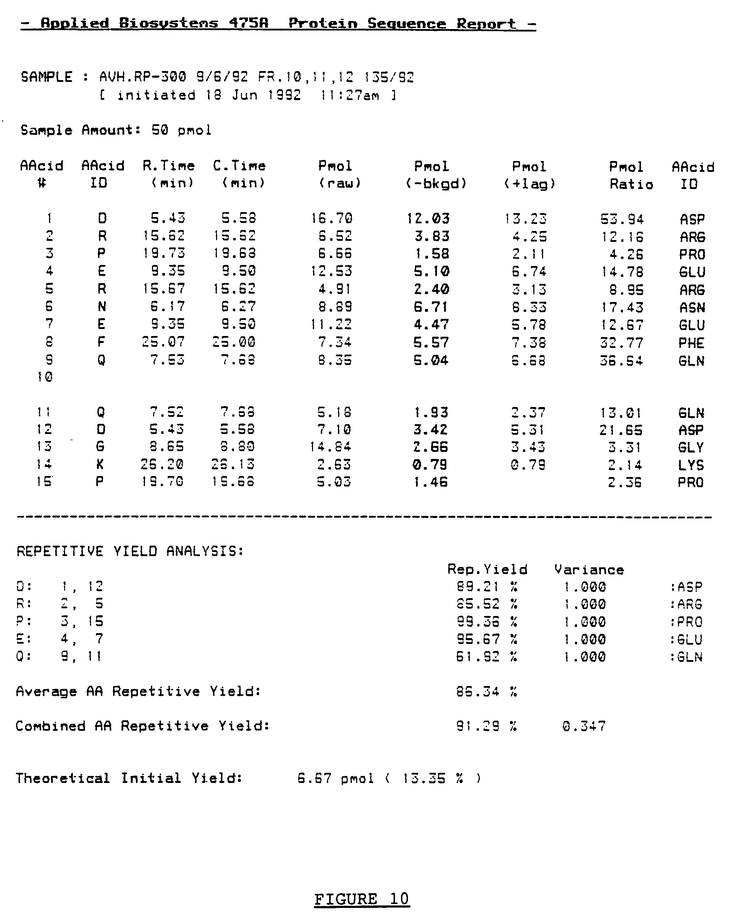

Aliquots (400µl) of fractions 10-12 from the last RP-HPLC step (see 2.7 in example 2) were pooled, concentrated by ultrafiltration and the concentrate (0.5µg) was subjected to microsequence analysis on model 475 protein microsequencer (Applied Biosystems, USA). The resulting sequence of the N-terminal 15 amino acid residues as identified by this system is given in fig. 10. The amino acid in cycle 10 was not identified while the Pro in cycle 3 was identified with relatively low confidence (given in Pmol ratio).

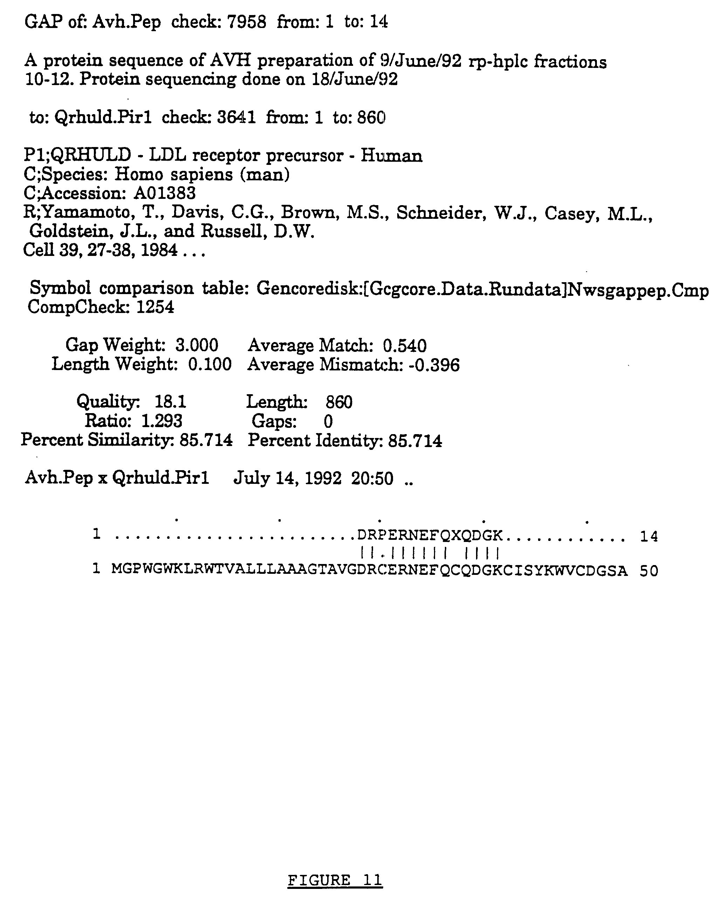

The resulting amino acid sequence was compared with NBRF protein databank and it gave 100% identity with the N-terminal sequence of the human low density lipoprotein (LDL) receptor starting at residue 25 of the precursor LDL receptor (residue 4 of mature LDL receptor). The amino acids in cycles 3, 10 and 15 could not be correctly identified by the sequencer since they are cystein residues which cannot be identified without preliminary modification. The identity between the isolated receptor and the known LDL receptor is shown in fig. 11.

The soluble antiviral protein of the present invention has a molecular weight of 29,000 or 40,000 according to the two methods that were used for its estimation. It is therefore concluded that the antiviral protein corresponds to the N-terminal cysteine-rich domain of the LDL receptor. According to Esser (Esser, V. et al. (1988) J.Biol.Chem. 263, 13282-13290) the cysteine-rich N-terminal domain of 292 amino acid residues is the ligand binding domain of LDL receptor and its calculated molecular weight is about 33,000-38,000 depending on the extent of glycosylation.

### Example 4: Immunoaffinity chromatography of the antiviral factor

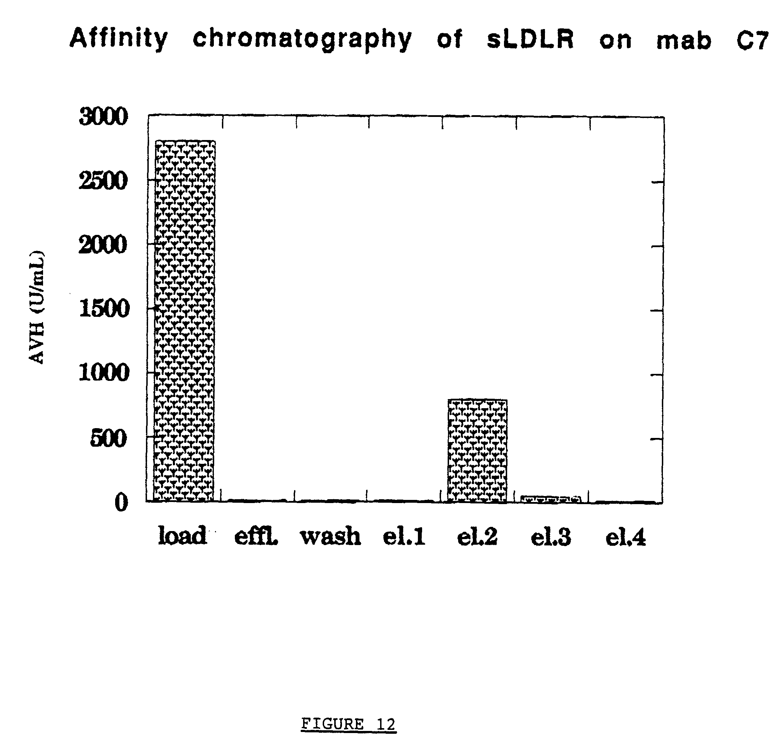

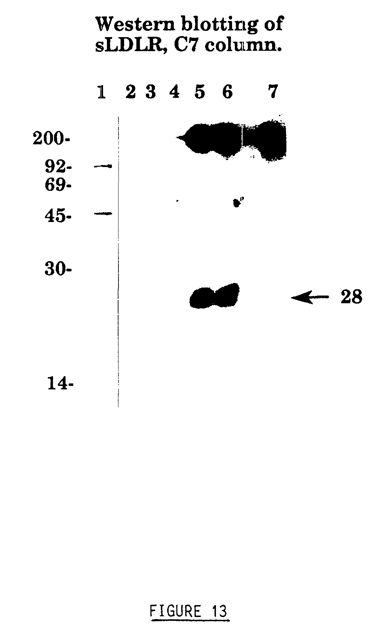

The most direct proof of identity of the antiviral factor and sLDLR was obtained by affinity chromatography of the crude antiviral protein on a monoclonal antibody C7 column. This antibody is directed against the ligand binding domain of bovine and human LDLR (Beisiegel, U. et al. (1981) J.Biol.Chem., 256, 11923-11931). Hybridoma C7 (ATCC, CRL 1691) was grown as ascites in mice and immunoglobulin was isolated from the ascitic fluid by ammonium sulfate fractionation. The C7 immunoglobulin (19mg), was coupled to 1ml agarose. Partially purified AVH from the 200mM NaCl fraction of the TSK-DEAE step ("load", 14ml, 37.8mg protein, 2800 units) was loaded on the column; the effluent ("effl.") was collected and the column was washed ("wash") with 70ml of 0.5M NaCl in phosphate buffered saline (PBS) followed by PBS (30ml). The column was then eluted with 50mM Na₂CO₃ (pH 11) (el.2 and el.3) with a recovery of 32% (Figure 12). The amount of protein in the eluted fractions was .15mg and the degree of purification was 83. SDS-PAGE and silver staining gave many bands. However, on Western blotting (Figure 13), with mab C7 (gel run under non-reducing conditions) no receptor was detected in the load, effluent and wash fractions (lanes 2, 3 and 4, respectively). However, the 28K band of the soluble LDL receptor was obtained in el.2 and el.3, (lanes 5 and 6). Also higher molecular weight bands were seen in el.2 and el.3, including weak 40K and 100K bands which probably are larger extracellular fragments of the LDL receptor. The strong band near 200K is probably monoclonal antibody C7 which leaked from the column and reacted with protein A, as it was identical with a C7 sample (lane 7).

### Example 5: Induction of cell surface LDLR by Interferons

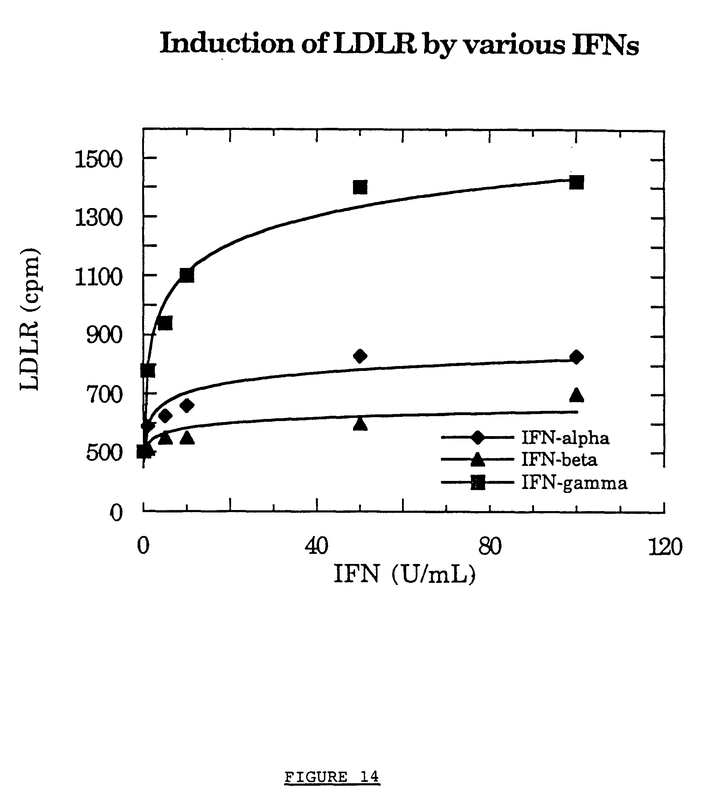

The ability of interferon to induce soluble LDL receptor suggests that it can also induce cell surface LDL receptor, since both receptor forms share the same gene. To study the possible induction of the full size LDL receptor WISH cells grown to confluence in 1.7cm wells were incubated with various interferons in medium containing 2% fetal bovine serum. The cells were washed, incubated with monoclonal antibody C7 from 2 hr at 4°C, washed, incubated with ¹²⁵I-protein A (about 1.33 x 10³ Bq (80,000 dpm)) for 2 hr at 4°C, washed, harvested with trypsin and counted. In a time course experiment it was found that maximal induction of LDL receptor with interferon-gamma (100 U/ml) occurred between 5 and 23 hr. A dose response study was then performed by incubating WISH cells for 19 hr with different interferons. It was found that interferon gamma was the most potent inducer of LDL receptor and maximal induction was seem with 10-50 U/ml. Interferon alpha was a much weaker inducer while interferon beta probably didn't induce LRL receptor at all (Figure 14). The induction of LDL receptor as well as its basal level were abolished in the presence of the protein synthesis inhibitor cycloheximide.

## Claims

1. A process for the production of a protein exhibiting anti-viral activity, comprising treating a culture of interferon-inducible cells with an interferon, incubating the culture, harvesting the culture supernatant, and purifying the protein from fractions of the supernatant exhibiting antiviral activity.

2. The process according to claim 1, wherein the cells employed are those capable of entering into an antiviral state in response to an interferon.

3. The process according to claim 1 or 2, wherein the interferon is interferon-gamma.

4. The process according to claim 1, wherein human WISH cells are employed.

5. The process according to any one of claims 1 to 4, comprising the steps of:
a) growing human WISH cells in culture to confluency, inducing the cells with IFN-gamma in a serum-free medium and harvesting the culture supernatant;
b) concentrating said supernatant by e.g. ultrafiltration with a membrane of molecular weight cut off of about 10,000 Da;
c) subjecting said concentrated supernatant of step b) to anion exchange chromatography to obtain partially purified active fractions of the protein;
d) applying said partially purified active fractions from step (c) to chromatography on a hydroxyapatite column to obtain partially purified active fractions of the protein;
e) applying said partially purified active fractions from step (d) to anion exchange HPLC to obtain partially purified active fractions of the protein;
f) applying said partially purified active fractions from step (e) to hydrophobic interaction chromatography to obtain partially purified active fractions of the protein;
g) applying said partially purified active fractions from step (f) to reversed phase high pressure liquid chromatography (HPLC) at about neutral pH to obtain partially purified protein;
h) repeating step (g) to obtain homogenous protein, defined by its ability to inhibit the cytopathic effect (CPE) exerted by vesicular stomatitis virus (VSV) on human WISH cells.

6. The process according to claim 5 wherein the ion exchange chromatography of step c) is performed on a TSK®-DEAE column.

7. The process according to claim 5 wherein the reversed phase HPLC step d) is performed on an Aquapore® RP300 column.

8. The process according to claim 5, wherein one or more of the chromatographic steps is replaced by immunoaffinity chromatography on an anti LDL receptor monoclonal antibody column.

9. The process according to claim 8, wherein the monoclonal antibody is C7 (ATCC, CRL 1691) and the soluble receptor is eluted at a high pH.

10. A purified protein obtainable by the process of any one of claims 1 to 9, with the proviso that said protein is not the full-length LDL receptor.

11. The protein according to claim 10 being a soluble extracellular domain of the mature LDL receptor, its muteins and fused proteins, their salts, functional derivatives and active fractions.

12. The protein according to claim 11, which is purified to homogeneity with respect to proteinaceous impurities.

13. The protein according to claim 11 or 12, comprising the ligand binding domain of the mature LDL receptor.

14. The protein according to any one of claims 11 to 13, having an amino acid sequence corresponding to amino acid residue 4 to 292 of the mature LDL receptor.

15. The protein according to any one of claims 11 to 14 and including the amino acid sequence as shown in the following: ASP-ARG-PRO-GLU-ARG-ASN-GLU-PHE-GLN- ( ) -GLN-ASP-GLY-LYS-PRO, wherein "()" is an unidentified amino acid.

16. A DNA molecule encoding a protein according to any one of claims 10 to 15 having an amino acid sequence corresponding to the amino acid sequence of the ligand binding domain of the mature LDL receptor, with the proviso that the DNA molecule does not consist of nucleotides 1 to 323 of the human LDL receptor cDNA sequence.

17. The DNA molecule according to claim 16, encoding a soluble LDL receptor having an amino acid sequence corresponding to amino acid residue 4 to 292 of the mature LDL receptor.

18. The DNA molecule according to claim 16, encoding a soluble LDL receptor including the amino acid sequence as follows:

ASP-ARG-PRO-GLU-ARG-ASN-GLU-PHE-GLN- () -GLN-ASP-GLY-LYS-PRO.

19. The DNA molecule which hybridises to the DNA molecule of any one of claims 16 to 18 and encodes a soluble LDL receptor, with the proviso that the DNA molecule does not consist of nucleotides 1 to 323 of the human LDL receptor cDNA sequence.

20. An expression vector comprising a DNA molecule according to any one of claims 16 to 19.

21. A cell line transformed with an expression vector according to claim 20.

22. A process for the production of a recombinant soluble LDL receptor comprising culturing a transformed cell line according to claim 21, and isolating the soluble LDL receptor.

23. A pharmaceutical composition comprising a soluble LDL receptor, its muteins and fused proteins, their salts, functional derivatives and active fractions according to claim 11, optionally together with a pharmaceutically acceptable carrier.

24. A pharmaceutical composition according to claim 23, further comprising another antiviral agent.

25. A pharmaceutical composition according to claim 24, in which the further antiviral agent is an interferon.

## Patentansprüche

1. Verfahren zur Herstellung eines Proteins mit antiviraler Aktivität, umfassend das Behandeln einer Kultur von durch Interferon induzierbaren Zellen mit einem Interferon, das Inkubieren der Kultur, das Emten des Kulturüberstandes und das Reinigen des Proteins aus Fraktionen des Überstandes, die antivirale Aktivität aufweisen.

2. Verfahren gemäß Anspruch 1, bei dem die verwendeten Zellen solche sind, die in Reaktion auf ein Interferon in einen antiviralen Zustand übergehen können.

3. Verfahren gemäß Anspruch 1 oder 2, bei dem das Interferon Interferon-gamma ist.

4. Verfahren gemäß Anspruch 1, bei dem humane WISH-Zellen verwendet werden.

5. Verfahren gemäß einem der Ansprüche 1 bis 4, umfassend die Schritte:
a) Wachsenlassen von humanen WISH-Zellen in Kultur bis zur Konfluenz, Induzieren der Zellen mit IFN-gamma in einem serumfreien Medium und Ernten des Kulturüberstandes;
b) Konzentrieren des Überstandes durch z. B. Ultrafiltration mit einer Membran mit Ausschlußgrenze bei einem Molekulargewicht von etwa 10.000 Da;
c) Auftrennen des konzentrierten Überstandes aus Schritt (b) durch Anionenaustauschchromatographie, wodurch teilweise gereinigte aktive Fraktionen des Proteins erhalten werden;
d) Auftragen der teilweise gereinigten aktiven Fraktionen aus Schritt (c) in eine Chromatographie in einer Hydroxyapatit-Säule, wodurch teilweise gereinigte aktive Fraktionen des Proteins erhalten werden;
e) Auftragen der teilweise gereinigten aktiven Fraktionen aus Schritt (d) in eine Anionenaustausch-HPLC, wodurch teilweise gereinigte aktive Fraktionen des Proteins erhalten werden;
f) Auftragen der teilweise gereinigten aktiven Fraktionen aus Schritt (e) in eine auf hydrophober Wechselwirkung beruhende Chromatographie, wodurch teilweise gereinigte aktive Fraktionen des Proteins erhalten werden;
g) Auftragen der teilweise gereinigten aktiven Fraktionen aus Schritt (f) in eine Umkehrphasen-Hochleistungs-Flüssigkeitschromatographie (HPLC) bei etwa neutralem pH, wodurch teilweise gereinigtes Protein erhalten wird;
h) Wiederholen des Schrittes (g), wodurch homogenes Protein erhalten wird, das durch seine Fähigkeit definiert ist, den cytopathischen Effekt (CPE) des Vesicuiar-Stomatitis-Virus (VSV) auf humane WISH-Zellen zu hemmen.

6. Verfahren gemäß Anspruch 5, bei dem die lonenaustauschchromatographie in Schritt c) in einer TSK®-DEAE-Säule durchgeführt wird.

7. Verfahren gemäß Anspruch 5, bei dem die Umkehrphasen-HPLC in Schritt d) in einer Aquapore®-RP300-Säule durchgeführt wird.

8. Verfahren gemäß Anspruch 5, bei dem ein oder mehrere chromatographische Schritte durch eine Immunaffinitätschromatographie in einer Säule mit monoklonalem Antikörper gegen den LDL-Rezeptor ersetzt sind.

9. Verfahren gemäß Anspruch 8, bei dem der monoklonale Antikörper C7 (ATCC, CRL 1691) ist und der lösliche Rezeptor bei einem hohen pH eluiert wird.

10. Gereinigtes Protein, das mit dem Verfahren gemäß einem der Ansprüche 1 bis 9 erhältlich ist, mit der Maßgabe, dass es sich bei dem Protein nicht um den vollständigen (full-length) LDL-Rezeptor handelt.

11. Protein gemäß Anspruch 10, bei dem es sich um eine lösliche extrazelluläre Domäne des reifen LDL-Rezeptors handelt, seine Muteine oder Fusionsproteine, oder Salze, funktionelle Derivate oder aktive Fraktionen davon.

12. Protein gemäß Anspruch 11, das in Bezug auf Proteinverunreinigungen bis zur Homogenität gereinigt ist.

13. Protein gemäß Anspruch 11 oder 12, das die Liganden-Bindungsdomäne des reifen LDL-Rezeptors umfasst.

14. Protein gemäß einem der Ansprüche 11 bis 13, das eine Aminosäuresequenz aufweist, die den Aminosäureresten 4 bis 292 des reifen LDL-Rezeptors entspricht.

15. Protein gemäß einem der Ansprüche 11 bis 14, das die nachstehend angegebene Sequenz enthält: ASP-ARG-PRO-GLU-ARG-ASN-GLU-PHE-GLN- () -GLN-ASP-GLY-LYS-PRO, in der "()" für eine nicht identifizierte Aminosäure steht.

16. DNA-Molekül, das ein Protein gemäß einem der Ansprüche 10 bis 15 kodiert; welches eine der Aminosäuresequenz der Liganden-Bindungsdomäne des reifen LDL-Rezeptors entsprechende Aminosäuresequenz aufweist, mit der Maßgabe, dass das DNA-Molekül nicht aus den Nukleotiden 1 bis 323 der cDNA-Sequenz des humanen LDL-Rezeptors besteht.

17. DNA-Molekül gemäß Anspruch 16, das einen löslichen LDL-Rezeptor kodiert, der eine den Aminosäureresten 4 bis 292 des reifen LDL-Rezeptors entsprechende Aminosäuresequenz aufweist.

18. DNA-Molekül gemäß Anspruch 16, das einen löslichen LDL-Rezeptor kodiert, der die folgende Aminosäuresequenz enthält:

ASP-ARG-PRO-GLU-ARG-ASN-GLU-PHE-GLN-()-GLN-ASP-GLY-LYS-PRO.

19. DNA-Molekül, das mit dem DNA-Molekül gemäß einem der Ansprüche 16 bis 18 hybridisiert und einen löslichen LDL-Rezeptor kodiert, mit der Maßgabe, dass das DNA-Molekül nicht aus den Nukleotiden 1 bis 323 der cDNA-Sequenz des humanen LDL-Rezeptors besteht.

20. Expressionsvektor, der ein DNA-Molekül gemäß einem der Ansprüche 16 bis 19 umfasst.

21. Zelllinie, die mit einem Expressionsvektor gemäß Anspruch 20 transformiert ist.

22. Verfahren zur Herstellung eines rekombinanten löslichen LDL-Rezeptors, umfassend das Kultivieren einer transformierten Zelllinie gemäß Anspruch 21 und das Isolieren des löslichen LDL-Rezeptors.

23. Pharmazeutische Zusammensetzung, umfassend einen löslichen LDL-Rezeptor, seine Muteine oder Fusionsproteine, oder Salze, funktionelle Derivate oder aktive Fraktionen davon gemäß Anspruch 11, fakultativ zusammen mit einem pharmazeutisch annehmbaren Träger.

24. Pharmazeutische Zusammensetzung gemäß Anspruch 23, die zusätzlich ein weiteres antivirales Agens enthält.

25. Pharmazeutische Zusammensetzung gemäß Anspruch 24, in der das weitere antivirale Agens ein Interferon ist.

## Revendications

1. Procédé de production de protéine présentant une activité anti-virale, comprenant le traitement d'une culture de cellules inductibles par l'interféron avec un interféron, l'incubation de la culture, la récolte du surnageant de la culture, et la purification de la protéine à partir de fractions de surnageant présentant une activité antivirale.

2. Procédé selon la revendication 1, dans lequel les cellules employées sont celles capables d'entrer en état antiviral en réponse à un interféron.

3. Procédé selon la revendication 1 ou 2, dans lequel l'interféron est l'interféron-gamma.

4. Procédé selon la revendication 1, dans lequel des cellules humaines WISH sont employées.

5. Procédé selon l'une quelconque des revendications 1 à 4, comprenant les étapes consistant à :
a) croître en culture des cellules humaines WISH à confluence, induire les cellules avec l'IFN-gamma dans un milieu sans sérum et récolter le surnageant de la culture ;
b) concentrer ledit surnageant par exemple par ultrafiltration sur une membrane à seuil de rétention d'environ 10000 Da ;
c) soumettre ledit surnageant concentré de l'étape b) à une chromatographie d'échange d'anions pour obtenir des fractions actives partiellement purifiées de la protéine ;
d) appliquer lesdites fractions actives partiellement purifiées de l'étape (c) à une chromatographie sur colonne d'hydroxyapatite pour obtenir des fractions actives partiellement purifiées de la protéine ;
e) appliquer lesdites fractions actives partiellement purifiées de l'étape (d) à une HPLC d'échange d'anions pour obtenir des fractions actives partiellement purifiées de la protéine ;
f) appliquer lesdites fractions actives partiellement purifiées de l'étape (e) à une chromatographie d'interactions hydrophobes pour obtenir des fractions actives partiellement purifiées de la protéine ;
g) appliquer lesdites fractions actives partiellement purifiées de l'étape (f) à une chromatographie liquide haute pression (HPLC) en phase inverse à pH proche de la neutralité pour obtenir la protéine partiellement purifiée;
h) répéter l'étape g) pour obtenir une protéine homogène, définie par sa capacité à inhiber l'effet cytopathique (ECP) exercé par le virus de stomatite vésiculaire (VSV) sur les cellules humaines WISH.

6. Procédé selon la revendication 5, dans lequel la chromatographie d'échange d'ions de l'étape c) est réalisée sur une colonne TSK®-DEAE.

7. Procédé selon la revendication 5, dans lequel l'HPLC en phase inverse de l'étape d) est réalisée sur une colonne Aquapore® RP300.

8. Procédé selon la revendication 5, dans lequel une ou plusieurs des étapes chromatographiques est remplacée par une chromatographie d'immunoaffinité sur une colonne d'anticorps monoclonal anti-récepteur de LDL.

9. Procédé selon la revendication 8, dans lequel l'anticorps monoclonal est C7 (ATCC, CRL 1691) et le récepteur soluble est élue à pH élevé.

10. Protéine purifiée susceptible d'être obtenue par le procédé selon l'une quelconque des revendications 1 à 9, à la condition que la dite protéine ne soit pas le récepteur LDL de taille complète.

11. Protéine selon la revendication 10, qui est un domaine extracellulaire soluble du récepteur de LDL mature, ses mutéines et protéines fusionnées, leurs sels, dérivés fonctionnels et fractions actives.

12. Protéine selon la revendication 11, qui est purifiée à homogénéité relativement aux impuretés protéiques.

13. Protéine selon la revendication 11 ou 12, comprenant le domaine de liaison au ligand du récepteur de LDL mature.

14. Protéine selon l'une quelconque des revendications 11 à 13, de séquence d'acides aminés correspondant aux résidus d'acides aminés 4 à 292 du récepteur de LDL mature.

15. Protéine selon l'une quelconque des revendications 11 à 14 et comprenant la séquence d'acides aminés suivante : 
ASP-ARG-PRO-GLU-ARG-ASN-GLU-PHE-GLN-()-GLN-ASP-
GLY-LYS-PRO,
 dans laquelle "()" est un acide aminé non identifié.

16. Molécule d'ADN codant pour une protéine selon l'une quelconque des revendications 10 à 15, de séquence d'acides aminés correspondant à la séquence d'acides aminés du domaine de liaison au ligand du récepteur de LDL mature, à la condition que la molécule d'ADN ne consiste pas en les nucléotides 1 à 323 de la séquence de l'ADNc du récepteur de LDL humain.

17. Molécule d'ADN selon la revendication 16, codant pour un récepteur de LDL soluble de séquence d'acides aminés correspondant aux résidus d'acides aminés 4 à 292 du récepteur de LDL mature.

18. Molécule d'ADN selon la revendication 16, codant pour un récepteur de LDL soluble comprenant la séquence d'acides aminés suivante :

ASP-ARG-PRO-GLU-ARG-ASN-GLU-PHE-GLN-()-GLN-ASP-
GLY-LYS-PRO.

19. Molécule d'ADN qui s'hybride à la molécule d'ADN de l'une quelconque des revendications 16 à 18 et code pour un récepteur de LDL soluble, à la condition que la molécule d'ADN ne consiste pas en les nucléotides 1 à 323 de la séquence de l'ADNc du récepteur de LDL humain.

20. Vecteur d'expression comprenant une molécule d'ADN selon l'une quelconque des revendications 16 à 19.

21. Lignée cellulaire transformée avec un vecteur d'expression selon la revendication 20.

22. Procédé de production d'un récepteur de LDL soluble recombinant comprenant la culture d'une lignée cellulaire transformée selon la revendication 21, et l'isolement du récepteur de LDL soluble.

23. Composition pharmaceutique comprenant un récepteur de LDL soluble, ses mutéines et protéines fusionnées, leurs sels, dérivés, fonctionnels et fractions actives selon la revendication 11, facultativement avec un support pharmaceutiquement acceptable.

24. Composition pharmaceutique selon la revendication 23, comprenant en plus un autre agent antiviral.

25. Composition pharmaceutique selon la revendication 24, dans laquelle l'autre agent antiviral est un interféron.
